# EUROPÄISCHE PATENTSCHRIFT

(11) **EP 2 286 790 B1**
(45) Veröffentlichungstag und Bekanntmachung des Hinweises auf die Patenterteilung: **25.01.2017**
(21) Anmeldenummer: 09168323.5
(22) Anmeldetag: 20.08.2009
(51) Int. Cl.: A61K 8/31, A61K 8/92, A61Q 5/02, A61Q 19/10

(54) **Produkte mit messtechnisch nachgewiesener olfaktorischer Wirkung zur Beeinflussung der psycho-physiologischen Ausgangslage**
Products with technically proven olfactory effect for influencing a psycho-physiological initial situation
Produits dotés d'un effet olfactif approuvé selon la technique de mesure pour l'influence de la position de sortie psycho-physiologique

(43) Veröffentlichungstag der Anmeldung: 23.02.2011
(73) Patentinhaber: Kneipp GmbH, 97084 Würzburg (DE)
(72) Erfinder: Wohlfart, Rainer, 97320, Sulzfeld am Main (DE); Ebert, Rebecca, 97852, Schöllbrunn (DE)
(74) Vertreter: Keller, Günter

(56) Entgegenhaltungen:
- EP-A1- 0 815 850
- WO-A1-2004/006882
- WO-A1-2008/050084
- WO-A1-2009/090355
- DE-A1- 4 419 470
- DE-U1-202004 018 321
- US-A- 4 921 874
- US-A- 5 397 497
- US-A- 5 766 628
- US-A1- 2004 063 604
- US-B1- 6 268 333
- US-B1- 6 447 788

## Beschreibung

Die vorliegende Erfindung betrifft kosmetische Reinigungs- und Pflegezusammensetzungen vorzugsweise zum Baden. In dem Bereich der Körperpflege sind verschiedenste Formen von Badezusätzen bekannt. Zu den Badezusätzen zählen Badesalze, klassische Wirkbäder, fette Ölbäder sowie Schaumbäder und Emulsionsbäder. Bekannt sind auch mehrphasige Zubereitungen, die in Ruhe erkennbar zweiphasig sind, durch kräftiges Schütteln homogenisiert werden können und sich nach einiger Zeit wieder abtrennen. Derartige Ölbäder weisen einen relativ hohen Anteil an verschiedenen Ölbestandteilen auf, die vor allem der Pflege der Haut dienen sollen.

EP 0 815 850 offenbart Badeölzusammensetzungen enthaltend verschiedene Öle und verhältnismäßig große Mengen an Ethanol.

US-PS 5,766,628 beschreibt im Wesentlichen wasserfreie ölige Bad- und Duschzusammensetzungen mit verschiedenen Tensiden sowie Efeuextrakt, Eukalyptusöl oder Pinienöl, wobei der Anteil an Öl 20 bis 90 Gew.-% der Zusammensetzung beträgt.

US-PS 6,447,788 offenbart Körperpflegemittel zum Baden, Duschen oder für die Haarpflege, die 40 bis 60 Gew.-% eines Tensids und 1 bis 5 Gew.-% eines Öls enthalten.

US 5,397,497 offenbart Badeöle, die Tenside, Solubilisatoren und verschiedene Öle, bevorzugt Piniennadelöl, enthalten.

Das deutsche Gebrauchsmuster 20 2004 018 321 offenbart Körperpflegepräparate zum Baden, Duschen oder für die Haarwäsche, die einen Honiganteil von wenigstens 40 Gew.-% neben einem Tensidanteil von wenigstens 40 Gew.-% aufweisen.

US-PS 4,921,874 beschreibt medizinische Badeöle, die 68 bis 95 Gew.-% an physiologisch annehmbaren Ölen und 5 bis 30 Gew.-% Emulgator enthalten. Bei den Ölen handelt es sich um Sojaöl oder Erdnussöl.

Gegenstand der US 6,268,333 ist eine beruhigende Geruchszusammensetzung enthaltend 1,3-dimethoxy-5-methylbenzol und eine Jasminzubereitung.

Die vorliegende Reinigungs- und Pflegezusammensetzung ist der Kategorie der klassischen Wirkbäder zuzuordnen, die anteilig fette Öle, ätherische Öle und Solubilisatoren aufweisen.

Bei bekannten Reinigungs- und Pflegezusammensetzungen wird häufig großer Wert auf die Reinigungsleistung der Zusammensetzung und/oder auf pflegende Aspekte des Badezusatzes gelegt.

Aufgabe der vorliegenden Erfindung ist es, eine Reinigungs- und Pflegezusammensetzung bereitzustellen, die gute Reinigungsleistung verbunden mit hoher Hautpflegequalität bereitstellt und darüber hinaus positiv die Stimmung der die Reinigungs- und Pflegezusammensetzungen verwendenden Person in gewünschter Weise beeinflusst. Erreicht wird dies durch die Zugabe eines oder mehrerer ätherischer Öle und/oder einzelner natürlicher, naturidentischer oder synthetischer Duftstoffe in verhältnismäßig hoher Konzentration. Bei der Bereitstellung des Badewassers wird die Reinigungs- und Pflegezusammensetzung aufgelöst. Ebenso werden bei der Aufbringung der Zusammensetzung beim Duschen die erfindungsgemäß eingesetzten ätherischen Öle freigesetzt. Diese können dabei von der die Reinigungs- und Pflegezusammensetzung verwendenden Person olfaktorisch über die Geruchsorgane aufgenommen werden.

Diese Aufnahme erfolgt unter folgenden Voraussetzungen:
1. Sinneseindrücke werden als erstes in einer Struktur im Gehirn verarbeitet, die sich Thalamus nennt und als "Tor zum Bewusstsein" bezeichnet wird. Nicht so Gerüche. Die Nervenimpulse, die in den olfaktorischen Sinneszellen ausgelöst werden, gelangen auf direktem Wege in das so genannte Limbische System. Dieses System bildet eine wichtige Grundlage unserer Gefühle. Gerüche können daher unmittelbar Gefühle auslösen, ohne dass diese Sinneseindrücke bewusst daher unmittelbar Gefühle auslösen, ohne dass diese Sinneseindrücke bewusst verarbeitet werden müssten. Die entstandenen Gefühle gehen in der Regel mit körperlichen Veränderungen, d. h. physiologischen Reaktionen, einher. Je nach Gefühl wird beispielsweise der Herzschlag schneller bzw. langsamer, oder die Hände fangen an zu schwitzen.
2. Stechende oder beißende Aromen wie die von Meerrettich, Alkohol oder Tabak erregen noch eine andere Art von Wahrnehmungssystem: die freien Nervenendigungen der Nervus Trigeminus. Dieses System hat Verbindungen zu einer Struktur, die Wachheit, physiologische Erregung und Aktivierung steuert: die im Hirnstamm befindliche Formatio reticularis. Die Funktionsweise dieses Systems machte man sich z. B. mit der Verwendung von Riechsalzen zur Erweckung Ohnmächtiger zunutze.
3. Wirkstoffe aus der Atemluft werden über die Nasenschleimhaut und die Bronchialschleimhaut aufgenommen, und gelangen so in den Blutkreislauf. Dadurch können sie Effekte auf innere Organe ausüben, und auch nach Durchquerung der Blut-Hirn-Schranke auf das Zentrale Nervensystem wirken.

Gegenstand der vorliegenden Erfindung sind daher eine Kosmetische Reinigungs- und Pflegezusammensetzung vorzugsweise Badezusätze mit einer die psycho-physiologische Ausgangslage beeinflussenden Wirkung, die 30-70 Gew.-% eines Solubilisators, Tensids oder einer Mischung verschiedener Tenside, und 0,1-20 Gew.-% einer Mischung mehrerer verschiedener ätherischer Öle und/oder einzelner natürlicher, naturidentischer oder synthetischer Duftstoffe enthält. Hierbei handelt es sich um ein Gemisch aus von Zitronen und Melisse gewonnenem ätherischem Öl, in Kombination mit Terpenen und Terpenoiden und Litsea Cubeba Öl.

In bevorzugter Ausführungsform enthält die Zusammensetzung zwischen 35 und 55 Gew.-% eines Solubilisators, Tensids oder einer Mischung verschiedener Tenside sowie 1,5-10 Gew.-% des ätherischen Öls oder einer Mischung mehrerer verschiedener ätherischer Öle und/oder einzelner natürlicher, naturidentischer oder synthetischer Duftstoffe.

Als weiteren Bestandteil enthält die Reinigungs- und Pflegezusammensetzung 0,1-5 Gew.-%, bevorzugt 1-2 Gew.-% eines weiteren Pflanzenöls, beispielsweise Sonnenblumenöl, Olivenöl oder Arganöl.

Weiterhin kann die Zusammensetzung 1-20 Gew.-%, bevorzugt 2-15 Gew.-% Glycerin enthalten. Das Glycerin wirkt einerseits als Viskositätsregler der fertigen Zusammensetzung und hat andererseits eine pflegende Wirkung auf die Haut.

Zur Stabilisierung der in der Reinigungs- und Pflegezusammensetzung verwendeten fetten und ätherischen Öle kann die Zusammensetzung in bevorzugter Ausführungsform bis 0,1% auf die zu stabilisierende Ölphase an Mischtocopherolkonzentrat 70% (natürlich) enthalten.

In bevorzugter Ausführungsform enthält die Reinigungs- und Pflegezusammensetzung keine Konservierungsstoffe, sowie keine Paraffine, Silikone und/oder Mineralöle.

In bevorzugter Ausführungsform enthält die Reinigungs- und Pflegezusammensetzung weiterhin einen oder mehrere Pflanzenextrakte. Geeignete Pflanzenextrakte stammen vom Hopfen, Baldrian, Melisse, Lavendel und/oder Rosmarin. Die Pflanzenextrakte werden in bevorzugter Ausführungsform durch CO₂-Extraktion gewonnen.

Manche Bestandteile, die üblicherweise in Reinigungs- und Pflegezusammensetzungen eingearbeitet werden, wie beispielsweise Detergenzien, stammen von tierischen Produkten her. So werden aus tierischem Fett, Talg, Knochen oder Haut durch chemische Behandlung waschaktive Substanzen, wie Seifen, hergestellt. Die erfindungsgemäße Reinigungs- und Pflegezusammensetzung besteht jedoch nur aus Komponenten, die aus pflanzlichen Produkten hergestellt wurden. Dabei enthält die Reinigungs- und Pflegezusammensetzung in bevorzugter Ausführungsform keine Bestandteile, die von Tieren herstammen.

Die erfindungsgemäße Reinigungs- und Pflegezusammensetzung beinhaltet als eine Hauptkomponente waschaktiven Substanzen Solubilisatoren oder Tenside in einer Menge von 30-70 Gew.-% bezogen auf die fertige Zusammensetzung. Tenside sind amphiphile Stoffe, die organische, unpolare Substanzen im Wasser lösen können. Bedingt durch ihren spezifischen Molekülaufbau mit mindestens einem hydrophilen und einem hydrophoben Molekülteil sorgen sie für eine Herabsetzung der Oberflächenspannung des Wassers, die Benetzung der Haut, die Erleichterung der Schmutzentfernung und Schmutzablösung, ein leichtes Abspülen und die Schaumverstärkung und Schaumregulierung.

Der hydrophile Anteil eines Tensidmoleküls wird meist durch polare funktionelle Gruppen, die von entsprechenden Säureresten herstammen, gebildet, wie beispielsweise -COO⁻, -OSO₃²⁻, -SO₃⁻. Der hydrophobe Teil des Tensids wird in der Regel durch unpolare Kohlenwasserstoffreste gebildet. Nach Art und Ladung des hydrophilen Molekülteils werden Tenside unterschieden nach anionischen Tensiden, amphoteren Tensiden bzw. nichtionischen Tensiden und kationischen Tensiden.

Im Rahmen der vorliegenden Erfindung werden bevorzugt anionische Tenside eingesetzt, wie Acyl-Isothionate, Alkylarylsulphonate, Alkylsulphonate, Sulphosuccinate sowie Schwefelsäureester, wie Alkylethersulfate oder Arylsulfate.

Vorteilhaft einzusetzende amphotere Tenside sind beispielsweise Acyl-/Dialkylethylendiamin. Als nichtionische Tenside werden bevorzugt eingesetzt Alkohole, Alkanolamide, Ester, die durch Veresterung von Carbonsäuren mit Ethylenoxid, Glycerin, Sorbitan oder anderen Alkoholen entstehen, sowie Ether, insbesondere ethoxylierte/propoxylierte Alkohole, ethoxylierte/propoxylierte Ester, ethoxylierte/propoxylierte Glycerinester. In besonders bevorzugter Ausführungsform wird als Hauptkomponente der waschaktiven Substanzen erfindungsgemäß Polyoxyethylen-Sorbitanmonolaureat (Tween 20) eingesetzt.

Neben der waschaktiven Komponente enthält die erfindungsgemäße Reinigungs- und Pflegezusammensetzung einen maßgeblichen Anteil des ätherischen Öls oder eines Gemisches mehrerer verschiedener ätherischer Öle und/oder einzelner natürlicher, naturidentischer oder synthetischer Duftstoffe. Die erfindungsgemäß eingesetzten ätherischen Öle sind sowohl natürliche Produkte als auch naturidentische Nachstellungen von Hauptkomponenten ätherischer Öle, die direkt aus Pflanzen gewonnen werden. Aufgrund der Herstellung der ätherischen Öle aus Pflanzen sind die erfindungsgemäß eingesetzten ätherischen Öle immer Gemische verschiedener chemischer Verbindungen, die hinsichtlich der einzelnen Komponenten und des Verhältnisses dieser Komponenten zueinander beeinflusst werden durch die Herkunft der Pflanzen und durch die genauen Bedingungen des Reinigungsverfahrens.

Zum großen Teil sind einzelne Komponenten der ätherischen Öle bekannt. Ätherische Öle werden hauptsächlich aus Pflanzen bzw. bestimmten Pflanzenteilen gewonnen. In chemischer Hinsicht unterscheiden sich die einzelnen Komponenten wesentlich voneinander. Es handelt sich bei den Bestandteilen der ätherischen Öle häufig um Kohlenwasserstoffe, Alkohole, Aldehyde, Ketone, Ester, Phenole, Phenylester und auch Verbindungen mit anderen komplexen Strukturen. Die Molekulargewichte der einzelnen Verbindungen sind relativ niedrig. Meistens überschreitet das Molekulargewicht nicht einen Wert zwischen 200 bis 300 Dalton.

Das folgende ätherische Öl wird erfindungsgemäß eingesetzt:
Das Öl ist ein Gemisch aus von Zitronen und Melisse gewonnenem ätherischem Öl, in Kombination mit Terpenen und Terpenoiden und Litsea Cubeba Öl. Die Verwendung eines derartigen Öls hat einen positiven Einfluss und hebt die Stimmungslage nachweislich.

Zitronenöl kann aus der Schale von Citrusfrüchten gewonnen werden. Zitronenöl kann bis zu 95 % Monoterpenkohlenwasserstoffe, hauptsächlich Limonen, aber auch andere Terpene, wie α-Terpinen und β-Pininen enthalten. Auch hier hängt das Verhältnis der einzelnen Komponenten zueinander von der Zitronenart und dem Ursprungsland ab.

In bevorzugter Ausführungsform wird auch Citronellaöl eingesetzt. Dieses wird aus verschiedenen aromatischen Gräsern durch Wasserdampfdestillation gewonnen. Es gibt verschiedene Typen von Citronellaöl, in Abhängigkeit vom Ursprungsland und der Art der Herstellung. Das Ceylon-Citronellaöl wird durch Dampfdestillation von frischen Blättern und Stengeln des Grases *Cymbopogon nardus* erhalten. Das Java-Citronellaöl wird durch Wasserdampfdestillation von Stengeln und Blättern des Grases *Cymbopogon winterianus* vor allem in Südosten Asiens, in Indien und in Indonesien gewonnen. Citronellaöl enthält vor allem Citronellal, Geraniol, Citronellol, Granylacetat, Citronellylacetat und viele andere Komponenten in geringeren Anteilen.

Litsea Cubebaöl ist das durch Wasserdampfdestillation aus den Früchten gewonnene ätherische Öl des Litseabaums (Litsea Cubeba Persoon). Die Pflanze hat zitronenartig duftende Blätter und Blüten und zählt zu den Lorbeergewächsen. Die kleinen Früchte des Baumes sehen aus wie Pfefferfrüchte, daher der volkstümliche Name Cubebenpfeffer. Das ätherische Öl enthält hauptsächlich Citral (70- 80%), Limonen (10 - 15%), Linalool, Geraniol, Nerol und wirkt aktivierend erfrischend und konzentrationsfördernd.

Einzelriechstoffe die sowohl natürlichen, naturidentischen oder synthetischen Ursprungs sein können, können die Duftwirkung der ätherischen Öle je nach Ihren spezifischen Eigenschaften verstärken, aber auch abschwächen. Entscheidend für Art und Intensität des Einflusses auf die psycho-physiologische Ausgangslage ist daher stets der olfaktorische Effekt der sich aus dem qualitativen und quantitativen Zusammenspiel aller vorhandenen Duftstoffe ergibt.

Die wesentlichen Bestandteile der erfindungsgemäßen kosmetischen Reinigungs- und Pflegezusammensetzung sind in der nachfolgenden Tabelle 1 zusammengestellt, wobei der bevorzugte und der besonders bevorzugte Bereich in Gew.-% angegeben sind. Die Gewichtsprozente sind als (Gewicht/Gewicht) angegeben und beziehen sich auf die fertige Zusammensetzung. Die Zusammensetzung wird immer mit gereinigtem Wasser auf 100 Gew.-% ergänzt.

**Tabelle 1**

| **Bestandteil** | **bevorzugter Bereich** | **besonders bevorzugter Bereich** |
|---|---|---|
| ätherische Öle, Mischungen ätherischer Öle und/oder einzelner natürlicher, naturidentischer oder synthetischer Duftstoffe | 0,001 - 15 | 1 - 10 |
| waschaktives Tensid | 35 - 75 | 40 - 60 |
| Pflanzenöl | 0 - 5 | 0,1 - 2 |
| Viskositätsregler (Glycerin) | 0 - 15 | 0 - 15 |
| Mischtocopherolkonzentrat | 0 - 0,1 | 0,01 - 0,1 |
| Farbstoff | 0 - 1,2 | 0,1 - 1,1 |
| Pflanzenextrakt | 0 - 1 | 0,01-1 |
| Wasser, gereinigt | ad 100 Gew.-% | ad 100 Gew.-% |

Bevorzugte Ausführungsformen werden in den nachfolgenden Beispielen wiedergegeben.

### Beispiel 1

| Handels-name | INCI/CTFA | Einwaage in Masse% | Verwendungszweck | Hersteller/Lieferant |
|---|---|---|---|---|
| Polysorbat 20 (Tween 20) | Polysorbate 20 | 50 - 60 | Solubilisator/ waschaktive Substanz | Evonik GmbH Goldschmidt GmbH, Goldschmidtstraße 100, 45127 Essen |
| Wasser, gereinigt | Aqua (Water) | 30 - 40 | Wasser | |
| Pö Freshness | Parfum (Fraganoe) | 5 - 10 | Olfaktorisch wirksamer Bestandteil | Firma Michael Schmitt Andernacher Str.8 50968 Köln |
| Sonnenblumenöl | Helianthus Anuus (Sunflower) Seed oil | 0,1 - 1 | Pflanzenöl | Gustav Heess GmbH, Am Gewerbering 4/6I, 83533 Edling |
| Farbstoff Chinolingelb | Chinolingelb CI 47005, E 104 | 0,1 - 1 | Farbstoff | Pharmorgana GmbH, An der Guldenmühle 7, 65817 Eppstein |
| Farbstoff gelb | FD& C yellow No. 5 CI 19140, E 140 | 0,01 - 0,1 | Farbstoff | BASF Chem Trade GmbH, Industriestr. 20, 91593 Burgbernhei m |
| Mischtocopherolkonzentr at 70% (natürlich) | Tocopherol | 0,01 - 0,1 | Stabilisator | Cognis Deutschland GmbH, Henkestraße 67, 40551 Düsseldorf |
| Summe | | 100 | | |

Dieser Badezusatz ruft bei der Anwendung eine messtechnisch nachgewiesene erfrischende, konzentrationsfördernde Wirkung und einen interessanten Eindruck hervor.

**Zusammensetzung Parfümöl (Pö) Freshness**

| Inhaltstoff | CAS-Nummer | Anteil in Gew% |
|---|---|---|
| Terpenes and Terpenoids, limonen fraction | 65996-98-7 | 10 - 50 |
| Linalyl acetate | 115-95-7 | 10 - 50 |
| Litsea Cubeba Fruit Oil | 68855-99-2 | 10 - 50 |
| Bois de Rose Bresil Essence | 8015-77-8 | 5 - 10 |
| Cornmint oil | 68917-18-0 | 5 - 10 |
| Linalool | 78 -70 6 | 5 - 10 |
| b-pinene | 127-91-3 | 1 - 5 |
| Alpha pinene | 80-56-8 | 0,1 - 1 |
| g-terpinene | 99-85-4 | 0,1 - 1 |
| Ocimene | 13877-91-3 | 0,1 - 1 |
| Spearmint Oil | 8008-79-5 | 0,1 - 1 |
| Terpinyl acetate | 80-26-2 | 0,1 - 1 |

### Beispiel 2 (Vergleich)

| Handels-name | INCI/CTFA | Enweage in % | Verwendungszweck | Hersteller/Lieferant |
|---|---|---|---|---|
| Polysorbat 20 (Tween 20) | Polysorbate 20 | 50-70 | Solubilisator/waschaktive Substanz | Evonik GmbH Goldschmidt GmbH, Goldschmidtstraße 100, 45127 Essen |
| Wasser, gereinigt | Aqua (Water) | 15-30 | Wasser | |
| Citronellöl Java 100% Stpfl. | Cymbopogon Nardus Citronella) Oil | 5-10 | olfaktorisch wirksamer Bestandteil | Düllberg Konzentra Obenhauptstr. 3D- 22335 Hamburg |
| Parfümöl Geranium | Parfum(Fragrance) | 2-5 | olfaktorisch wirksamer Bestandteil | Düllberg Konzentra Obenhauptstr. 3D- 22335 Hamburg |
| Parfümöl (II) | Parfum(Fragrance) | 2-5 | olfaktorisch wirksamer Bestandteil | Firma Michael Schmitt Andemacher Str.8 50968 Köln |
| Baldrianöl 25 % Stpfl. | Parfum(Fragrance), Valeriana Officinalis Oil | 2-5 | olfaktorisch wirksamer Bestandteil | Düllberg Konzentra Obenhauptstr. 3D- 22335 Hamburg |
| Hopfenextrakt 4-6:1 (OO2) | Humulus Lupulus Hops Extract | 0,1-1 | Pflanzenextrakt | Falvex Naturextrakte, Nordstraße 7D - 66780 Ehingen |
| Farbstoff blau (FD & C Blue No. 1, Ol 42090) | Ol 42090 (Blue 1) | 0,01-0,1 | Farbstoff | Pharmorgana GmbH, An der Guldenmühle 7, 65817 Eppstein |
| Mischtocopherolkonzentrat 70% (natürlich) | Tocopherol | 0,01-01 | Stabilisator | Cognis Deutschland GmbH, Henkestraße 67, 40551 Düsseldorf |
| | | 100,0 | | |

Dieser Badezusatz hat eine positive, entspannende Wirkung und einen beruhigenden Effekt. Er sollte vorzugsweise vor dem Schlafengehen angewendet werden.

**Zusammensetzung Parfümöl (II)**

| Inhaltstoff | CAS-Nummer | Anteil in Gew% |
|---|---|---|
| Eucalyptus globulus Leaf Oil | 8000-48-4 | 10 - 50 |
| Clove bud oil | 8000-34-8 | 10 - 50 |
| Lavendula Hybrida Oil | 8022-15-9 | 5 - 10 |
| Turpentine Oil | 9005-90-7 | 5 - 10 |
| Camphor | 21368-68-3 | 5 - 10 |
| Alpha-pinene | 80-56-8 | 5 - 10 |
| Alpha-terpineol | 98-55-5 | 1 - 5 |
| Camphene | 79-92-5 | 1 - 5 |
| dl-borneol | 507-70-0 | 1 - 5 |
| Linalyl acetate | 115-95-7 | 1 - 5 |
| p - Cymene | 99-87-6 | 1 - 5 |
| Shiu oil | 8022-91-1 | 1 - 5 |
| Thym blanc | | 1 - 5 |
| Thymol | 89-83-8 | 1 - 5 |
| b-pinene | 127-91-3 | 0,1 - 1 |
| Bornyl acetate | 76-49-3 | 0,1 - 1 |
| Dipentene | 138-86-3 | 0.1 - 1 |
| Isobornyl acetate | 125-12-2 | 0,1 - 1 |
| Linalool | 78-70-6 | 0,1 - 1 |
| Myrcene | 123-53-3 | 0,1 - 1 |
| Weitere Parfümbestandteile | | < 0,1 |

**Zusammensetzung Parfümöl "Geranium"**

| | | |
|---|---|---|
| **Citronellol** | | **50%** |
| **Citronellylformiat** | | **16%** |
| **Geraniol** | | **12%** |
| **Linalool** | | **5%** |
| **Weiter Parfümbestandteile** | | **Ad 100%** |

**Zusammensetzung Baldrianöl 25%Stpfl**

| | | |
|---|---|---|
| **Badrianöl** | | **25%** |
| **Andere ätherische Öle** | | **11%** |
| **Naturidentische Riechstoffe** | | **64%** |

### Beispiel 3

| Handels-name | INCI/CTFA | Einwaage in Masse% | Verwendungszweck | Hersteller/Lieferant |
|---|---|---|---|---|
| Pö Zitrone-Melisse | Parfum(Fragance) | 1-5 | olfaktorisch wirksamer Bestandteil | Firma Michael Schmitt Andemacher Str.8 50968 Köln |
| Sonnenblumenöl | Helianthus Anuus (Sunflower) Seed oil | 0,1-1 | Pflanzenöl | Gustav Heess GmbH, Am Gewerbering 4/6I, 83533 Eding |
| Glycerol 85% | Glycerin | 10-15 | Viskositätssenkender Hautpflegestoff | Fauth & Co GmbH, Innstraße 35-37, 68199 Mannheim |
| Polysorbat 20 (Tween 20) | Polysorbate 20 | 40-50 | Solubilisator/waschaktive Substanz | Evonik GmbH Goldschmidt GmbH, Goldschmidtstraße 100, 45127 Essen |
| Mischtocopherolkonzentr at 70% (natürlich) | Tocopherol | 0,01-0,1 | Stabilisator | Cognis Deutschland GmbH, Henkestraße 67, 40551 Düsseldorf |
| Farbstoff Chinolingelb | Chinolingelb CI 47005, E 104 | 0,1-1 | Farbstoff | Pharmorgana GmbH, An der Guldenmühle 7, 65817 Eppstein |
| Farbstoff gelb | FD&Cyellow No. 5 CI 19140, E 140 | 0,01-0,1 | Farbstoff | BASF Chem Trade GmbH, Industriestr. 20, 91593 Burgbernheim |
| Wasser, gereinigt | Aqua (Water) | 30-40 | Wasser | |
| Summe | | 100,0000 | | |

Dieser Badezusatz hat eine entspannende und stresslösende Wirkung. Er sollte bevorzugt nach körperlicher, seelischer oder emotionaler Anspannung angewendet werden.

**Zusammensetzung Parfümöl Zitrone-Melisse**

| Inhaltstoff | CAS-Nummer | Anteil in Gew% |
|---|---|---|
| Terpenes and Terpenoids, limonen fraction | 65996-98-7 | 10 - 50 |
| Citral | 5392-40-5 | 10 - 50 |
| Litsea Cubeba Fruit Oil | 90063-59-2 | 10 - 50 |
| Citronella Oil | 8000-29-1 | 5 - 10 |
| Lemon Oil | 8008-56-8 | 1 - 5 |
| Weitere Parfümbestandteile | | <1 |

### Beispiel 4 (Vergleichsbeispiel)

Wettbewerbsprodukt mit folgender Auslobung:
Gesundheitsbad
Wohltuende Entspannung vor dem Einschlafen.

Und setzt sich wie folgt zusammen:
INCI: Aqua, Sodium Laureth Sulfate, Polysorbate 20, Glycerin, PEG-6-Caprylic/Capric Glycerides, Parfum, Coco-Glucosides, Glyceryl Oleate, Valeriana officinalis, Lavandula angustifolia, Cymbopogon nardus, Humulus lupulus, Ethoxydiglycol, Propylene Glycol, Butylene Glycol, Lactic Acid, Glucose, Phenoxyethanol, Methylparaben, Butylparaben, Propylparaben, Isobutylparaben, Hexylcinnamal, Butyphenyl Methyprobional, Linalool, Limonene, Alpha-Isometyl lonone, Geraniol, Cl 42051

Um die Auswirkungen der Reinigungs-und Pflegezusammensetzung auf den Gemütszustand der Probanden objektiv messen zu können, wurden verschiedene Parameter bestimmt. Die Probanden erhielten zunächst eine Duftprobe der Zusammensetzung, dann wurden Veränderungen der Gesichtsmuskeln, Veränderungen der Herzschlagfrequenz, des Pulses und der Gehirnaktivität bestimmt.

Als physiologische Aktivierungs-Indikatoren sind für die Ermittlung der Ergebnisse folgende Parameter relevant:
- Die Anzahl der elektrodermalen Reaktionen (NS.SCR) und die mittlere Herzrate als Indikatoren einer valenzunabhängigen Aktivierung.
- Die Summenamplitude der Elektrodermalen Reaktionen als Indikator einer emotionalen Aktivierung.
- Die Modulation der Pulsvolumenamplitude (PVA) als Indikator für Interesse weckende Eigenschaften und eine Hinwendung zum Reiz.

Eine mit der Methodik des Objective Emotional Assessment (OEA) durchgeführte Produktevaluation liefert als Ergebnis objektiv-quantifizierte Aussagen über die wahrgenommenen Produkteigenschaften sowie die durch die Interaktion mit dem Produkt beim Konsumenten ausgelöste emotionale Reaktion. Auf Grundlage von empirisch unter kontrollierten Laborbedingungen erhobenen Daten wird für jedes Produkt das durch dieses erzeugte spezifische psychophysiologische Emotionsprofil bestimmt.

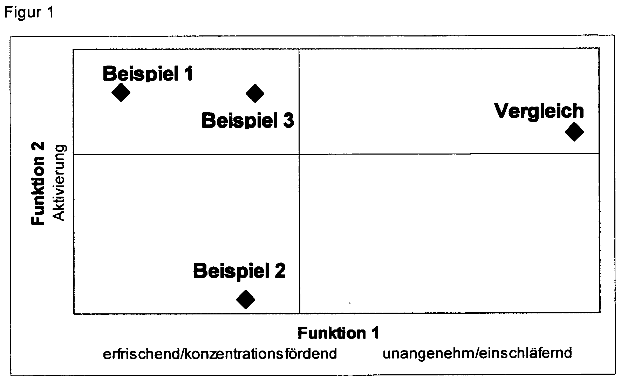

Die für die Untersuchung der Badeprodukte besonders relevanten Eigenschaften "Stimmungsaufhellung" und "Entspannung" lassen sich übersetzten in die beiden üblicherweise in der Psychologie zur Charakterisierung von emotionalen Reaktionen bzw. Zuständen verwendeten Dimensionen Valenz (angenehm - unangenehm) und Aktivierung (entspannt - angespannt). Die statistische Methode der Diskriminanzanalyse gestattet die Lage jedes getesteten Produktes auf diesen Dimensionen (d.h. die Ausprägung der durch das Produkt ausgelösten Empfindungen) aufgrund der spezifischen Ausprägungen der erfassten psychophysiologischen Emotionsindikatoren eindeutig zu lokalisieren und zufallskritisch abzusichern. Die untenstehende Graphik (Abbildung 1) veranschaulicht den emotionalen Raum, in dem die Lage der Produkte bestimmt wird. Paarweise Vergleiche der Produkte, bzw. die Bildung einer Rang-Reihenfolge aller Produkte bezogen auf die interessierenden Charakteristika sind ein zentraler Teil der Untersuchungsergebnisse.

Die besonderen Vorteile der verwendeten psychophysiologischen Methodik im Vergleich zu herkömmlichen (üblicherweise rein verbalen) Methoden der Produkttestung bestehen in der größeren Objektivität und geringeren Anfälligkeit für bewusste Verfälschung sowie in dem Potenzial auch automatisch ablaufende emotionale Reaktion valide abbilden zu können. Des weiteren wird durch die multivariante Erfassung unterschiedlicher Emotionskomponenten der holistische Wahrnehmungseindruck abgebildet. Im Vergleich zur selektiven Betrachtung einzelner, isolierter Produkteigenschaften berücksichtigt dieser Ansatz auch mögliche Interaktionen unterschiedlicher Eigenschaften, die in qualitativ andersartigen Wahrnehmungseindrücken resultieren können.

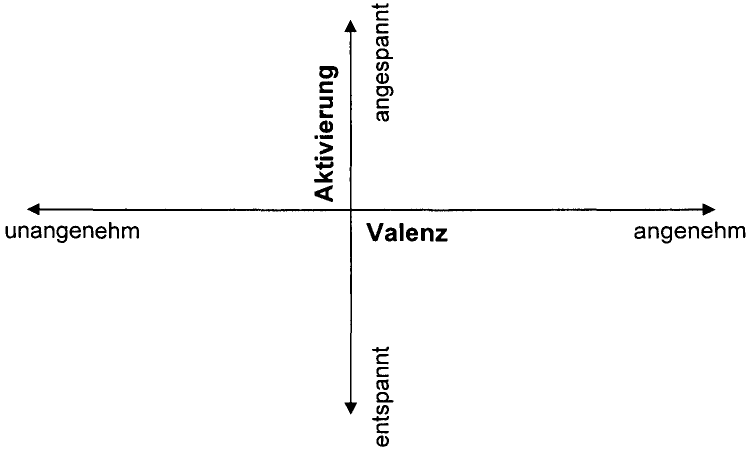

### Abbildung 1:

Emotionaler Raum zur Lokalisierung psychophysiologischer Profile unterschiedlicher Produkte

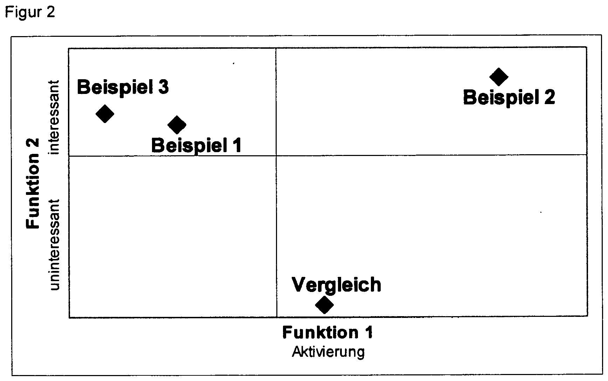

Mit Hilfe der oben näher dargelegten Methode wurden die Reinigungs- und Pflegezusammensetzung vorzugsweise Badezusätze gemäß Beispiel 1, 2 und 3 getestet und es wurde ein Vergleichstest mit einem Produkt gemäß Beispiel 4 durchgeführt. Dabei wurden zusammenfassend die in Figur 1 und 2 dargestellten Ergebnisse erhalten.
Figur 1 zeigt, dass die erfindungsgemäßen Beispiele alle eine erfrischende, konzentrationsfördernde Wirkung haben. Das Vergleichsbeispiel weist diese Wirkung dagegen nicht auf.
Figur 2 zeigt, dass sämtliche erfindungsgemäßen Zusammensetzungen einen interessanten Eindruck bei den Testpersonen hinterlassen, wohingegen das Vergleichsbeispiel einen uninteressanten und langweiligen Eindruck macht.

## Patentansprüche

1. Kosmetische Reinigungs- und/oder Pflegezusammensetzung mit messtechnisch nachgewiesener olfaktorischer Wirkung auf die psycho-physiologische Ausgangslage, die 30-70 Gew.-% mindestens eines Tensides und/oder eines Solubilisators, sowie 0,1-20 Gew.-% mindestens eines ätherischen Öls, einer Mischung mehrerer ätherischer Öle und/oder einzelner natürlicher, naturidentischer oder synthetischer Duftstoffe enthält, dadurch gekennnzeichnet, dass es sich bei diesen 0,1-20 Gew.-% um ein Gemisch aus von Zitronen und Melisse gewonnenem ätherischem Öl, in Kombination mit Terpenen und Terpenoiden und Litsea Cubeba Öl handelt.

2. Zusammensetzung nach Anspruch 1, **dadurch gekennzeichnet, dass** sie zwischen 35 und 55 Gew.-% eines Solubilisators, eines Tensids oder einer Mischung verschiedener Tenside enthält.

3. Zusammensetzung nach einem der Ansprüche 1 oder 2, **dadurch gekennzeichnet, dass** sie 1,5-10 Gew.-% der Mischung mehrerer verschiedener ätherischer Öle mit nachgewiesener olfaktorischer Wirkung enthält.

4. Zusammensetzung nach Anspruch 1, **dadurch gekennzeichnet, dass** das ätherische Öl die nachfolgend aufgeführten Inhaltsstoffe in den angegebenen relativen Anteilen enthält:
| Inhaltstoff | Anteil in Gew% |
|---|---|
| Terpene und Terpenoide, limonen fraction | 10 - 50 |
| Citral | 10 - 50 |
| Litsea Cubeba Fruit Oil | 10 - 50 |
| Citronella Oil | 5 - 10 |
| Lemon Oil | 1 - 5 |
| Weitere Parfümbestandteile | <1 |

5. Zusammensetzung nach einem der vorhergehenden Ansprüche, **dadurch gekennzeichnet, dass** sie 0,1-5 Gew.-% eines weiteren Pflanzenöls enthält.

6. Zusammensetzung nach einem der vorhergehenden Ansprüche, **dadurch gekennzeichnet, dass** sie 1-20 Gew.-% Glycerin enthält.

7. Zusammensetzung nach einem der vorhergehenden Ansprüche, **dadurch gekennzeichnet, dass** sie keine Konservierungsstoffe enthält.

8. Zusammensetzung nach einem der vorhergehenden Ansprüche, **dadurch gekennzeichnet, dass** sie keine Paraffine, Silikone und/oder Mineralöle enthält.

9. Zusammensetzung nach einem der vorhergehenden Ansprüche, **dadurch gekennzeichnet, dass** sie weiterhin einen oder mehrere Pflanzenextrakte enthält.

10. Zusammensetzung nach einem der vorhergehenden Ansprüche, **dadurch gekennzeichnet, dass** sie keine Bestandteile enthält, die von Tieren herstammen.

## Claims

1. A cosmetic cleaning and/or care composition having a metrological proven olfactory effect on the psycho-physiological initial situation and containing from 30-70% by weight of at least one surfactant, and/or of a solubilizer as well as from 0.1-20% by weight of at least one essential oil, a mixture of several essential oils and/or individual natural, natural-identical, or synthetic odorous substances, **characterized in that** the 0.1-20% by weight are a mixture of an essential oil recovered from lemons and melissa in combination with terpenes and terpenoids and Litsea Cubeba oil.

2. The composition according to claim 1, **characterized in that** it contains between 35 and 55% by weight of a solubilizer, a surfactant, or a mixture of various surfactants.

3. The composition according to any one of claims 1 or 2, **characterized in that** it contains from 1.5-10% by weight of the mixture of several different essential oils having a proven olfactory effect.

4. The composition according to claim 1, **characterized in that** the essential oil contains the active ingredients listed below in the given relative proportions:
| Ingredient | Proportion in % by weight |
|---|---|
| terpenes and terpenoids, limonen fraction | 10-50 |
| citral | 10-50 |
| Litsea Cubeba fruit oil | 10-50 |
| citronella oil | 5-10 |
| lemon oil | 1-5 |
| further perfume constituents | <1 |

5. The composition according to any one of the preceding claims, **characterized in that** it contains from 0.1-5% by weight of a further vegetable oil.

6. The composition according to any one of the preceding claims, **characterized in that** it contains from 1-20 % by weight of glycerol.

7. The composition according to any one of the preceding claims, **characterized in that** it does not contain any preservatives.

8. The composition according to any one of the preceding claims, **characterized in that** it does not contain any paraffins, silicones, and/or mineral oils.

9. The composition according to any one of the preceding claims, **characterized in that** it further contains one or more plant extracts.

10. The composition according to any one of the preceding claims, **characterized in that** it does not contain any constituents derived from animals.

## Revendications

1. Composition de nettoyage et/ou de soin cosmétique avec effet olfactif prouvé selon la technique de mesure sur la situation de départ psycho-physiologique, qui contient 30 à 70 % en poids d'au moins un tensioactif et/ou un agent de solubilisation, ainsi que 0,1 à 20 % en poids d'au moins une huile volatile, d'un mélange de plusieurs huiles volatiles et/ou de différentes senteurs naturelles, identiques aux naturelles ou synthétiques, **caractérisée en ce que** ces 0,1 à 20 % en poids sont un mélange d'huile volatile extraite du citron et de la mélisse, en combinaison avec des terpènes et des terpénoïdes et de l'huile de Litsea Cubeba.

2. Composition selon la revendication 1, **caractérisée en ce qu'**elle contient entre 35 et 55 % en poids d'un agent de solubilisation, d'un tensioactif ou d'un mélange de différents tensioactifs.

3. Composition selon l'une quelconque des revendications 1 ou 2, **caractérisée en ce qu'**elle contient 1,5 à 10 % en poids du mélange de plusieurs huiles volatiles différentes avec effet olfactif prouvé.

4. Composition selon la revendication 1, **caractérisée en ce que** l'huile volatile contient les composants cités ci-après dans les proportions relatives indiquées :
| Composant | Proportion en% en poids |
|---|---|
| Terpènes et terpénoïdes, fraction limonène | 10 - 50 |
| Citral | 10 - 50 |
| Huile du Fruit de Litsea Cubeba | 10 - 50 |
| Huile de Citronnelle | 5 - 10 |
| Huile de citron | 1 - 5 |
| Autres ingrédients de parfum | <1 |

5. Composition selon l'une quelconque des revendications précédentes, **caractérisée en ce qu'**elle contient 0,1 à 5 % en poids d'une autre huile végétale.

6. Composition selon l'une quelconque des revendications précédentes, **caractérisée en ce qu'**elle contient 1 à 20 % en poids de glycérine.

7. Composition selon l'une quelconque des revendications précédentes, **caractérisée en ce qu'**elle ne contient aucun conservateur.

8. Composition selon l'une quelconque des revendications précédentes, **caractérisée en ce qu'**elle ne contient aucun(e) paraffine, silicone et/ou huile minérale.

9. Composition selon l'une quelconque des revendications précédentes, **caractérisée en ce qu'**elle contient en outre un ou plusieurs extraits de plantes.

10. Composition selon l'une quelconque des revendications précédentes, **caractérisée en ce qu'**elle ne contient aucun composant d'origine animale.
